# EUROPEAN PATENT APPLICATION

(11) **EP 2 149 548 A1**
(43) Date of publication of application: **03.02.2010**
(21) Application number: 08761480.6
(22) Date of filing: 11.04.2008
(51) Int. Cl.: C07C 69/007, C07C 69/28, C07C 67/14, A61K 31/23, A61K 31/222, A61K 31/25

(54) **METHOD FOR THE CHEMICAL SYNTHESIS OF CAPSINOIDS**

(30) Priority: 24.04.2007 ES 200701099
(71) Applicant: Universidad De Cádiz, 11001 Cádiz (ES)
(72) Inventor: GONZÁLEZ MOLINILLO, José María, E-11510 Puerto Real (Cádiz) (ES); MACÍAS DOMÍNGUEZ, Francisco Antonio, E-11510 Puerto Real (Cádiz) (ES); VARELA MONTOYA, Rosa María, E-11510 Puerto Real (Cádiz) (ES); PALMA LOVILLO, Miguel, E-11510 Puerto Real (Cádiz) (ES); GARCÍA BARROSO, Carmelo, E-11510 Puerto Real (Cádiz) (ES); FERNÁNDEZ BARBERO, Gerardo, E-11510 Puerto Real (Cádiz) (ES)
(74) Representative: ABG Patentes, S.L.
(86) International application number: PCT/ES2008/000233
(87) International publication number: WO 2008/129094

(57) **Abstract**

Method for the chemical synthesis of capsinoids, natural compounds with proven biological activity, from vanillin, comprising the protection of the alcohol group thereof, reduction of the aldehyde to alcohol, esterification of same and deprotection of the first protected alcohol group. In this way, the desired compounds are generated with high purity and said compounds can be easily separated with no mixing owing to the competitive esterification on the aromatic ring.

## Description

### Field of the Invention

The invention belongs to the field of the chemical synthesis of natural bioactive compounds.

### Background of the Invention

Capsinoids are natural compounds found in different varieties of sweet peppers. Their greatest interest lies in the proven biological activity they have. The interest in capsinoids is so high that its patent application has been filed with possible uses in "healthy" drinks of the capsaicin and of the nordihydrocapsaicin (Patent No. WO 9944981 A1), in addition to its use in analgesics and in food (Patent No. JP 2001158738 A2).

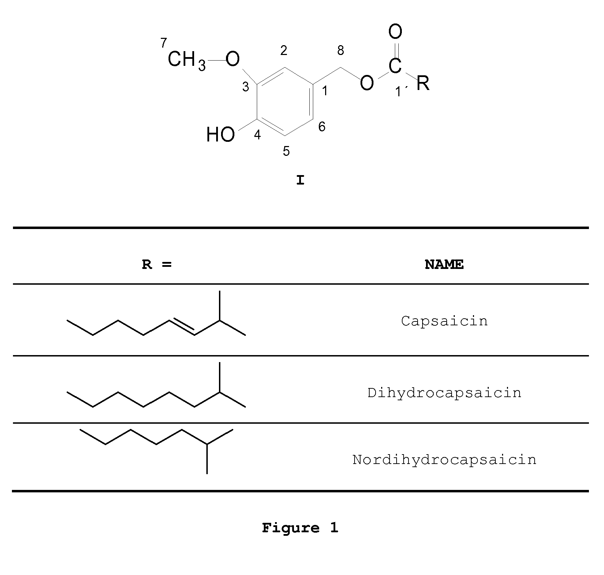

Chemically, capsinoids are esters formed from the condensation of the vanillyl alcohol and fatty acids with different chain length. The different known natural capsinoids can be obtained depending on the number of carbons of the side chain (R) or on if they have unsaturations or not (Figure 1).

Capsinoids are compounds presenting remarkable antioxidant properties (see, for example, Rosa, A.; Deiana, M.; Casu, V.; Paccagnini, S.; Appendino, G.; Ballero, M.; Dessi, M. A. J. Agric. Food Chem. 2002, 50, 7396-7401), are chemopreventive and anticancer compounds (see Macho, A.; Lucena, C.; Sancho, R.; Daddario, N.; Minassi, A.; Muñoz, E.; Appendino, G. Eur. J. Nutr. 2003, 42, 2-9), promote energy metabolism and suppress the accumulation of fats in the organism (see Ohnuki, K.; Niwa, S.; Maeda, S.; Inoue, N.; Yazawa, S.; Fushiki, T. Biosci. Biotechnol. Biochem. 2001, 65, 2033-2036) and are potent antiinflammatory compounds (see Sancho, R.; Lucena, C.; Macho, A.; Calzado, M. A.; Blanco-Molina, Minassi, M. A.; Appendino, G.; Muñoz, E. Eur. J. Immunol. 2002, 32, 1753-1763).

For this reason, the study of methods for the synthesis of both natural and synthetic capsinoids, the latter of which have properties similar to natural capsinoids, is of great interest due to the difficulty of isolating these compounds. In recent years several synthetic methodologies for this family of compounds have been developed.

Kobata *et al*. (see Kobata, K.; Todo, T.; Yazawa, S.; Iwai, K.; Watanabe, T. J. Agric. Food Chem. 1998, 46, 1695-1697) chemically synthesized 4-hydroxy-3-methoxybenzyl 8-methylnonanoate (dihydrocapsaicin). To that end vanillyl alcohol was reacted with the chloride of 8-methylnonanoic acid in pyridine. The reaction mixture was magnetically stirred for 2 hours at 0°C.

Appendino *et al.* (see Appendino, G.; Minassi, A.; Daddario, N.; Bianchi, F.; Tron, G.C. Org. Lett. 2002, 22. 3839-3841) subsequently studied the chemoselective esterification of phenolic acids with alcohols. This type of reaction has been applied for the synthesis of the vanillyl nonanoate. For the synthesis of vanillyl nonanoate, vanillyl alcohol was reacted with nonanoic acid, using tetrahydrofuran (THF) and equimolar amounts of diisopropyl azodicarboxylate (DIAD) and triphenylphosphine (TPP) as reaction medium. The reaction was carried out at room temperature and for 24 hours. In these conditions, the yield obtained for vanillyl nonanoate was 67%.

Recently, Torregiani *et al.* (see Torregiani, E.; Seu, G.; Minassi, A.; Appendino, G. Tetrahedron Lett. 2005, 46, 2193-2196) have developed a novel method for the selective esterification of phenol alcohols, the reaction being catalyzed by means of cerium(III) chloride. By means of this novel method for synthesis, vanillyl nonanoate was obtained with a yield of 70%.

### Description of the Invention

The invention consists of a novel general, selective and simple method for the synthesis of capsinoids. This method consists of 4 reaction steps starting from vanillin (4-hydroxy-3-methoxybenzaldehyde).

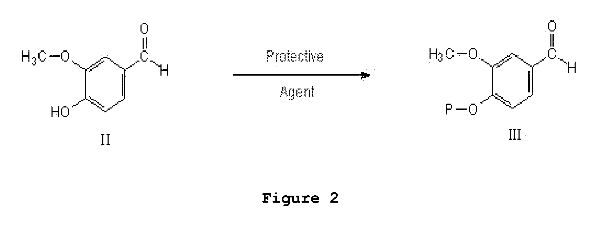

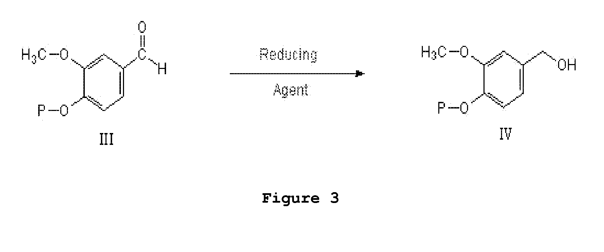

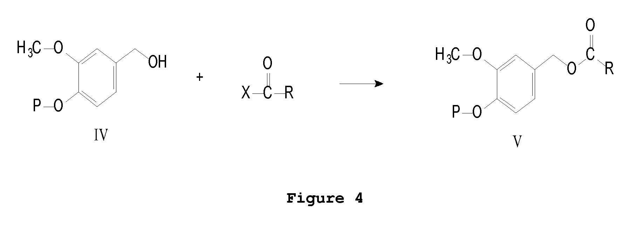

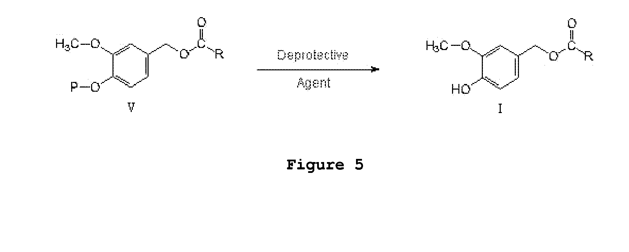

The method starts from vanillin, which has an aldehyde group in the position which is to be esterified. This allows protecting the aromatic hydroxyl, blocking it efficiently by means of a protective group. The subsequent reduction of the aldehyde group gives rise to a protected vanillyl alcohol which does not have selectivity problems in its esterification. Once this is done, it can be deprotected generating the desired compounds with high purity and said compounds can be easily separated with no mixing owing to the competitive esterification on the aromatic ring. In brief, the method can be defined in:
Step 1: Protection of the hydroxyl group of the vanillin (Figure 2).
Step 2: Reduction of the carbonyl of the protected vanillin (Figure 3).
Step 3: Esterification of the reduced and protected vanillin with acyl chlorides or any other acylating agent (anhydrides, dialkyl azodicarboxylate derivatives, etc.) (Figure 4).
Step 4: Deprotection of the protected capsinoids (Figure 5).

These four reaction steps comprise high-yield selective reactions.

### Brief Description of the Drawings

Figure 1: Base structure of the capsinoids and side chains of known natural capsinoids.
Figure 2: Step 1 of the method.- Protection of the hydroxyl group of the vanillin (P is a protective group).
Figure 3: Step 2 of the method.- Reduction of the carbonyl of the protected vanillin.
Figure 4: Step 3 of the method.- Esterification of the reduced and protected vanillin with acyl chlorides or any other acylating agent (anhydrides, dialkyl azodicarboxylate derivatives, etc.).
Figure 5: Step 4 of the method.- Deprotection of the protected capsinoids.

### Embodiment of the Invention

### Example: Synthesis of capsinoids using t-butyldimethylsilyl ethers as protective group and acyl chlorides as acylating agent.

### Step 1: Silylating 4-hydroxy-3-methoxybenzaldehyde in the aromatic hydroxyl.

This reaction step (Figure 2) consists of protecting the hydroxyl group of vanillin (4-hydroxy-3-methoxy-benzaldehyde) to prevent that subsequent esterifications occur in this position of the molecule.

The starting material for the synthesis of capsinoids is vanillin. The hydroxyl group of vanillin is protected with t-butyl-dimethylsilyl chloride, to subsequently carry out a reduction of the aldehyde group to be able to esterify in this position and thus introduce the side chains of the capsinoids.

The vanillin (5.8995 g, 0.0388 mol) (II) is introduced in a 250 mL round-bottom flask and dissolved in 30 mL of anhydrous pyridine. 1.2 equivalents of t-butyl-dimethylsilane chloride (7.0128 g, 0.0466 mol) are added to this solution. The reaction mixture is maintained under magnetic stirring at room temperature and in inert argon atmosphere for 24 hours.

The reaction is followed by TLC (eluent: 20% ethyl acetate, 80% hexane; stain: anisaldehyde). Once the reaction has ended, it is stopped with ethyl acetate (100 mL). The organic phase (ethyl acetate) is washed several times with a concentrated CuSO₄x5H₂O aqueous solution to remove the pyridine, until the color change of the CuSO₄x5H₂O solution is not observed.

The organic phase is dried with anhydrous MgSO₄, filtered and concentrated under vacuum to remove the ethyl acetate (room temperature). Finally a yellowish precipitate (III) corresponding to silylated vanillin (4-tert-butyldimethylsilyloxy-3-methoxybenzaldehyde) is obtained (yield: 98%).

### Characterizing 4-tert-butyldimethylsilyloxy-3-methoxybenzaldehyde:

*4-tert-butyldimethylsilyloxy-3-methoxybenzaldehyde*: ¹H-NMR (CDCl₃, 399.945 MHz): δ 7.35 (1H, d, 1.8 Hz, H-2), δ 7.32 (1H, dd, 1.8, 8.1 Hz, H-6), δ 6.91 (1H, d, 8.1 Hz, H-5), δ 3.81 (3H, s, H-7), δ 9.74 (1H, s, H (ald.)), δ 0.14 (6H, s, 2xCH₃), δ 0.95 (9H, s, 3xCH₃). ¹³C-NMR (CDCl₃, 100.576 MHz): δ 151.2 (s, C-4), δ 151.4 (s, C-3), δ 110.0 (d, C-2), δ 130.8 (s, C-1), δ 126.0 (d, C-6), δ 120.5 (d, C-5), δ 55.3 (q, C-7), δ 190.8 (d, C-8), δ 18.3 (s, C-9), δ -4.7 (2C, q, C-10), δ 25.4 (3C, q, C-11).

### Step 2: Reducing the carbonyl of 4-tert-butyldimethylsilyloxy-3-methoxybenzaldehyde.

The following reaction step (Figure 3) consists of reducing the carbonyl group of silylated vanillin (III) (4-tert-butyldimethylsilyloxy-3-methoxybenzaldehyde) to its corresponding alcohol (IV). The reducing agent used was diisobutylaluminum hydride (1M in toluene) (DIBAL).

Silylated vanillin (III) (3.3518 g, 0.0125 mol) is dissolved in 40 mL of anhydrous tetrahydrofuran. Subsequently 2 equivalents of diisobutylaluminum hydride (1M in Toluene) are slowly added in an ice bath and the reaction mixture is maintained under magnetic stirring, at room temperature and in inert argon atmosphere for 48 hours.

The reaction is followed by TLC (eluent: 20% ethyl acetate, 80% hexane; stain: anisaldehyde). Once the reaction has taken place, it is stopped with water. The aqueous phase is extracted 3 times with ethyl acetate (3x100 mL). The organic phase is dried with anhydrous MgSO₄, filtered and concentrated under reduced pressure to remove the ethyl acetate (room temperature). Finally, an impure dark brown oil is obtained. This oil is dissolved in a small amount of ethyl acetate and silica gel is added to obtain the head of the separation column. The head of the column is dried under reduced pressure (room temperature).

Chromatographic separation is carried out with silica gel and the polarity of the eluent is 10% ethyl acetate in hexane. Chromatographic separation is followed by TLC (eluent: 20% ethyl acetate, 80% hexane; stain: anisaldehyde). Finally a dark brown oil (IV) corresponding to the reduced and silylated vanillin (4-tert-butyldimethylsilyloxy-3-methoxybenzyl alcohol) is obtained (yield: 74%). This oil is used as a starting material for the synthesis of all silylated capsinoids.

### Characterizing 4-tert-butyldimethylsilyloxy-3-methoxybenzyl alcohol:

*4-tert-butyldimethylsilyloxy-3-methoxybenzyl alcohol*: ¹H-NMR (CDCl₃, 399.945 MHz): δ 6.82 (1H, d, 1.8 Hz, H-2), δ 6.72 (1H, dd, 1.8, 8.2 Hz, H-6), δ 6.76 (1H, d, 8.2 Hz, H-5), δ 3.74 (3H, s, H-7), δ 4.50 (2H, s, H-8), δ 2.65 (1H, s, OH), δ 0.11 (6H, s, 2xCH₃), δ 0.96 (9H, s, 3xCH₃). ¹³C-NMR (CDCl₃, 100.576 MHz): δ 150.8 (s, C-4), δ 144.3 (s, C-3), δ 111.0 (d, C-2), δ 134.4 (s, C-1), δ 120.6 (d, C-6), δ 119.3 (d, C-5), δ 55.2 (q, C-7), δ 64.9 (t, C-8), δ 18.3 (s, C-9), δ -4.8 (2C, q, C-10), δ 25.6 (3C, q, C-11).

### Step 3: Esterifying 4-tert-butyldimethylsilyloxy-3-methoxybenzyl alcohol with acyl chlorides

This reaction step consists of esterifying the reduced and protected vanillin (IV) (4-tert-butyldimethylsilyloxy-3-methoxybenzyl alcohol) with the corresponding acyl chlorides, according to the capsinoid which is to be synthesized (Figure 4).

The reduced and protected vanillin (IV) (0.8442g) is dissolved in 15 mL of anhydrous pyridine in a 50 mL round-bottom flask. Inert argon atmosphere is introduced. Next, the corresponding acyl chloride (2 equivalents) is slowly added to this solution and it is left under stirring for 18 hours. The reaction is followed by TLC (eluent: 20% ethyl acetate, 80% hexane; stain: anisaldehyde).

Once the reaction has ended, it is stopped with ethyl acetate. The organic phase is washed 3 times with 10% HCl to remove the pyridine from the medium (3x50 mL). Subsequently, the organic phase is filtered and dried with anhydrous MgSO₄ and concentrated under reduced pressure (T ≈ 30°C). A brownish-gray oil is obtained, which is dissolved in a small amount of ethyl acetate, then silica gel is added to obtain the head of the separation column. The ethyl acetate is dried under reduced pressure (T ≈ 30°C) to obtain the head of the column.

Chromatographic separation is carried out with silica gel and the polarity of the eluent is 20% ethyl acetate in hexane. The chromatographic separation is followed by TLC (eluent: 20% ethyl acetate, 80% hexane; stain: anisaldehyde). Finally a yellowish oil (O2C-O12C) and a pale yellowish precipitate (O13CO16C) corresponding to silylated capsinoids (V) are obtained (yields 84-99%).

### Characterizing synthesized silylated capsinoids:

*4-tert-butyldimethylsilyloxy-3-methoxybenzyl ethanoate*: ¹H-NMR (CDCl₃, 399.945 MHz): δ 6.84 (1H, s^{b}, H-2), δ 6.81 (1H, d^{b}, H-6), δ 6. 81 (1H, d^{b}, H-5), δ 3.80 (3H, s, H-7), δ 5.01 (2H, s, H-8), δ 0.14 (6H, s, 2xCH₃), δ 0.98 (9H, s, 3xCH₃), δ 2.08 (3H, s, H-2'). ¹³C-NMR (CDCl₃, 100.576 MHz): δ 150.9 (s, C-4), δ 145.2 (s, C-3), δ 112.6 (d, C-2), δ 129.2 (s, C-1), δ 121.2 (d, C-6), δ 120.8 (d, C-5), δ 55.4 (q, C-7), δ 66.4 (t, C-8), δ 18.4 (s, C-9), δ -4.6 (2C, q, C-10), δ 25.7 (3C, q, C-11), δ 170.9 (s, C-1'), δ 21.1 (q, C-2').

*4-tert-butyldimethylsilyloxy-3-methoxybenzyl propanoate*: ¹H-NMR (CDCl₃, 399.945 MHz): δ 6.84 (1H, s^{b}, H-2), δ 6.80 (1H, d^{b}, H-6), δ 6. 80 (1H, d^{b}, H-5), δ 3.80 (3H, s, H-7), δ 5.03 (2H, s, H-8), δ 0.14 (6H, s, 2xCH₃), δ 0.98 (9H, s, 3xCH₃), δ 2.36 (2H, q, 7.6 Hz, H-2'), δ 1.15 (3H, t, 7.6 Hz, H-3'). ¹³C-NMR (CDCl₃, 100.576 MHz): δ 150.9 (s, C-4), δ 145.1 (s, C-3), δ 112.5 (d, C-2), δ 129.4 (s, C-1), δ 121.1 (d, C-6), δ 120.7 (d, C-5), δ 55.4 (q, C-7), δ 66.2 (t, C-8), δ 18.4 (s, C-9), δ -4.7 (2C, q, C-10), δ 25.7 (3C, q, C-11), δ 174.3 (s, C-1'), δ 27.6 (t, C-2'), δ 9.1 (q, C-3').

*4-tert-butyldimethylsilyloxy-3-methoxybenzyl butanoate*: ¹H-NMR (CDCl₃, 399.945 MHz) : δ 6.84 (1H, s^{b}, H-2), δ 6.80 (1H, d^{b}, H-6), δ 6. 80 (1H, d^{b}, H-5), δ 3.78 (3H, s, H-7), δ 5.02 (2H, s, H-8), δ 0.14 (6H, s, 2xCH₃), δ 0.98 (9H, s, 3xCH₃), δ 2.31 (2H, t, 7.6 Hz, H-2'), δ 1.65 (2H, tq, 7.6, 7.2 Hz, H-3'), δ 0.92 (3H, t, 7.2 Hz, H-4'). ¹³C-NMR (CDCl₃, 100.576 MHz) : δ 150.8 (s, C-4), δ 145.0 (s, C-3), δ 112.4 (d, C-2), δ 129.5 (s, C-1), δ 121.0 (d, C-6), δ 120.6 (d, C-5), δ 55.3 (q, C-7), δ 66.0 (t, C-8), δ 18.3 (s, C-9), δ -4.7 (2C, q, C-10), δ 25.6 (3C, q, C-11), δ 174.3 (s, C-1'), δ 36.1 (t, C-2'), δ18.3 (t, C-3'), δ 13.5 (q, C-4').

*4-tert-butyldimethylsilyloxy-3-methoxybenzyl pentanoate*: ¹H-NMR (CDCl₃, 399.945 MHz): δ 6.82 (1H, s^{b}, H-2), δ 6.79 (1H, d^{b}, H-6), δ 6. 79 (1H, d^{b}, H-5), δ 3.77 (3H, s, H-7), δ 5.00 (2H, s, H-8), δ 0.13 (6H, s, 2xCH₃), δ 0.97 (9H, s, 3xCH₃), δ 2.31 (2H, t, 7.6 Hz, H-2'), δ 1.60 (2H, quin, 7.6, 7.6 Hz, H-3'), δ 1.31 (2H, tq, 7.6, 7.2 Hz, H-4'), δ 0.87 (3H, t, 7.2 Hz, H-5'). ¹³C-NMR (CDCl₃, 100.576 MHz): δ 151.1 (s, C-4), δ 145.3 (s, C-3), δ 112.7 (d, C-2), δ 129.8 (s, C-1), δ 121.3 (d, C-6), δ 120.9 (d, C-5), δ 55.6 (q, C-7), δ 66.3 (t, C-8), δ 18.6 (s, C-9), δ -4.5 (2C, q, C-10), δ 25.9 (3C, q, C-11), δ 173.8 (s, C-1'), δ 34.2 (t, C-2'), δ 27.2 (t, C-3'), δ 22.4 (t, C-4'), δ 13.9 (q, C-5').

*4-tert-butyldimethylsilyloxy-3-methoxybenzyl hexanoate*: ¹H-NMR (CDCl₃, 399.945 MHz) : δ 6.82 (1H, s^{b}, H-2), δ 6.79 (1H, d^{b}, H-6), δ 6.79 (1H, d^{b}, H-5), δ 3.77 (3H, s, H-7), δ 5.01 (2H, s, H-8), δ 0.13 (6H, s, 2xCH₃), δ 0.97 (9H, s, 3xCH₃), δ 2.31 (2H, t, 7.6 Hz, H-2'), δ 1.61 (2H, quin, 7.6, 7.6 Hz, H-3'), δ 1.27 (2H, m, 7.6, 7.2 Hz, H-4'), δ 1.27 (2H, m, 7.2, 6.8 Hz, H-5'), δ 0.85 (3H, t, 6.8 Hz, H-6'). ¹³C-NMR (CDCl₃, 100.576 MHz): δ 150.8 (s, C-4), δ 145.0 (s, C-3), δ 112.4 (d, C-2), δ 129.4 (s, C-1), δ 121.0 (d, C-6), δ 120.6 (d, C-5), δ 55.3 (q, C-7), δ 66.0 (t, C-8), δ 18.3 (s, C-9), δ -4.8 (2C, q, C-10), δ 25.6 (3C, q, C-11), δ 173.5 (s, C-1'), δ 34.2 (t, C-2'), δ 24.5 (t, C-3'), δ 31.2 (t, C-4'), δ 22.2 (t, C-5'), δ 13.8 (q, C-6').

*4-tert-butyldimethylsilyloxy-3-methoxybenzyl heptanoate*: ¹H-NMR (CDCl₃, 399.945 MHz): δ 6.83 (1H, s^{b}, H-2), δ 6.79 (1H, d^{b}, H-6), δ 6.79 (1H, d^{b}, H-5), δ 3.78 (3H, s, H-7), δ 5.01 (2H, s, H-8), δ 0.13 (6H, s, 2xCH₃), δ 0.97 (9H, s, 3xCH₃), δ 2.32 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 0.85 (3H, t, 6.8 Hz, H-7'). ¹³C-NMR (CDCl₃, 100.576 MHz) : δ 150.8 (s, C-4), δ 145.0 (s, C-3), δ 112.4 (d, C-2), δ 129.4 (s, C-1), δ 121.1 (d, C-6), δ 120.7 (d, C-5), δ 55.3 (q, C-7), δ 66.1 (t, C-8), δ 18.3 (s, C-9), δ -4.7 (2C, q, C-10), δ 25.6 (3C, q, C-11), δ 173.6 (s, C-1'), δ 34.3 (t, C-2'), δ 24.8 (t, C-3'), δ 28.7 (t, C-4'), δ 31.3 (t, C-5'), δ 22.4 (t, C-6'), δ 13.9 (q, C-7').

*4-tert-butyldimethylsilyloxy-3-methoxybenzyl octanoate*: ¹H-NMR (CDCl₃, 399.945 MHz) : δ 6.82 (1H, s^{b}, H-2), δ 6.78 (1H, d^{b}, H-6), δ 6.78 (1H, d^{b}, H-5), δ 3.77 (3H, s, H-7), δ 5.00 (2H, s, H-8), δ 0.12 (6H, s, 2xCH₃), δ 0.97 (9H, s, 3xCH₃), δ 2.31 (2H, t, 7.6 Hz, H-2'), δ 1.61 (2H, quin, 7.6, 7.3 Hz, H-3'), δ 1.25 (2H, m, H-4'), δ 1.25 (2H, m, H-5'), δ 1.25 (2H, m, H-6'), δ 1.25 (2H, m, H-7'), δ 0.85 (3H, t, 6.8 Hz, H-8'). ¹³C-NMR (CDCl₃, 100.576 MHz) : δ 150.8 (s, C-4), δ 145.0 (s, C-3), δ 112.4 (d, C-2), δ 129.4 (s, C-1), δ 121.1 (d, C-6), δ 120.6 (d, C-5), δ 55.3 (q, C-7), δ 66.0 (t, C-8), δ 18.3 (s, C-9), δ -4.8 (2C, q, C-10), δ 25.6 (3C, q, C-11), δ 173.6 (s, C-1'), δ 34.3 (t, C-2'), δ 24.9 (t, C-3'), δ 29.0 (t, C-4'), δ 28.8 (t, C-5'), δ 31.5 (t, C-6'), δ 22.5 (t, C-7'), δ 13.9 (q, C-8').

*4-tert-butyldimethylsilyloxy-3-methoxybenzyl nonanoate*: ¹H-NMR (CDCl₃, 399.945 MHz) : δ 6.82 (1H, s^{b}, H-2), δ 6.78 (1H, d^{b}, H-6), δ 6.78 (1H, d^{b}, H-5), δ 3.77 (3H, s, H-7), δ 5.00 (2H, s, H-8), δ 0.12 (6H, s, 2xCH₃), δ 0.97 (9H, s, 3xCH₃), δ 2.31 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 0.85 (3H, t, 6.8 Hz, H-9'). ¹³C-NMR (CDCl₃, 100.576 MHz) : δ 150.8 (s, C-4), δ 145.0 (s, C-3), δ 112.4 (d, C-4), δ 129.7 (s, C-1), δ 121.0 (d, C-6), δ 120.6 (d, C-5), δ 55.3 (q, C-7), δ 66.0 (t, C-8), δ 18.3 (s, C-9), δ -4.8 (2C, q, C-10), δ 25.6 (3C, q, C-11), δ 173.6 (s, C-1'), δ 34.3 (t, C-2'), δ 24.9 (t, C-3'), δ 29.0 (t, C-4'), δ 29.1 (t, C-5'), δ 29.0 (t, C-6'), δ 31.7 (t, C-7'), δ 22.5 (t, C-8'), δ 13.9 (q, C-9').

*4-tert-butyldimethylsilyloxy-3-methoxybenzyl decanoate*: ¹H-NMR (CDCl₃, 399.945 MHz) : δ 6.82 (1H, s^{b}, H-2), δ 6.78 (1H, d^{b}, H-6), δ 6.78 (1H, d^{b}, H-5), δ 3.77 (3H, s, H-7), δ 5.01 (2H, s, H-8), δ 0.13 (6H, s, 2xCH₃), δ 0.97 (9H, s, 3xCH₃), δ 2.31 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 0.85 (3H, t, 6.8 Hz, H-10'). ¹³C-NMR (CDCl₃, 100.576 MHz): δ 150.8 (s, C-4), δ 145.0 (s, C-3), δ 112.3 (d, C-2), δ 129.4 (s, C-1), δ 121.0 (d, C-6), δ 120.6 (d, C-5), δ 55.3 (q, C-7), δ 66.0 (t, C-8), δ 18.3 (s, C-9), δ -4.8 (2C, q, C-10), δ 25.6 (3C, q, C-11), δ 173.6 (s, C-1'), δ 34.2 (t, C-2'), δ 24.9 (t, C-3'), δ 29.0 (t, C-4'), δ 29.2 (t, C-5'), δ 29.3 (t, C-6'), δ 29.2 (t, C-7'), δ 31.7 (t, C-8'), δ 22.5 (t, C-9'), δ 14.0 (q, C-10').

*4-tert-butyldimethylsilyloxy-3-methoxybenzyl undecanoate*: ¹H-NMR (CDCl₃, 399.945 MHz): δ 6.83 (1H, s^{b}, H-2), δ 6.80 (1H, d^{b}, H-6), δ 6.80 (1H, d^{b}, H-5), δ 3.79 (3H, s, H-7), δ 5.02 (2H, s, H-8), δ 0.14 (6H, s, 2xCH₃), δ 0.98 (9H, s, 3xCH₃), δ 2.32 (2H, t, 7.6 Hz, H-2'), δ 1.62 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 0.86 (3H, t, 6.8 Hz, H-11'). ¹³C-NMR (CDCl₃, 100.576 MHz): δ 150.9 (s, C-4), δ 145.1 (s, C-3), δ 112.4 (d, C-2), δ 129.5 (s, C-1), δ 121.1 (d, C-6), δ 120.7 (d, C-5), δ 55.4 (q, C-7), δ 66.1 (t, C-8), δ 18.4 (s, C-9), δ -4.7 (2C, q, C-10), δ 25.6 (3C, q, C-11), δ 173.7 (s, C-1'), δ 34.3 (t, C-2'), δ 24.9 (t, C-3'), δ 29.1 (t, C-4'), δ 29.3 (t, C-5'), δ 29.5 (t, C-6'), δ 29.4 (t, C-7'), δ 29.2 (t, C-8'), δ 31.8 (t, C-9'), δ 22.6 (t, C-10'), δ 14.1 (q, C-11').

*4-tert-butyldimethylsilyloxy-3-methoxybenzyl dodecanoate*: ¹H-NMR (CDCl₃, 399.945 MHz): δ 6.82 (1H, s^{b}, H-2), δ 6.78 (1H, d^{b}, H-6), δ 6.78 (1H, d^{b}, H-5), δ 3.77 (3H, s, H-7), δ 5.02 (2H, s, H-8), δ 0.14 (6H, s, 2xCH₃), δ 0.98 (9H, s, 3xCH₃), δ 2.31 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 0.86 (3H, t, 6.8 Hz, H-12'). ¹³C-NMR (CDCl₃, 100.576 MHz) : δ 150.8 (s, C-4), δ 145.0 (s, C-3), δ 112.4 (d, C-2), δ 129.5 (s, C-1), δ 121.1 (d, C-6), δ 120.7 (d, C-5), δ 55.4 (q, C-7), δ 66.1 (t, C-8), δ 18.4 (s, C-9), δ -4.7 (2C, q, C-10), δ 25.6 (3C, q, C-11), δ 173.7 (s, C-1'), δ 34.3 (t, C-2'), δ 24.9 (t, C-3'), δ 29.1 (t, C-4'), δ 29.3 (t, C-5'), δ 29.5 (t, C-6'), δ 29.4 (t, C-7'), δ 29.3 (t, C-8'), δ 29.2 (t, C-9'), δ 31.8 (t, C-10'), δ 22.6 (t, C-11'), δ 14.0 (q, C-12').

*4-tert-butyldimethylsilyloxy-3-methoxybenzyl tridecanoate*: ¹H-NMR (CDCl₃, 399.945 MHz): δ 6.82 (1H, s^{b}, H-2), δ 6.77 (1H, d^{b}, H-6), δ 6.77 (1H, d^{b}, H-5), δ 3.76 (3H, s, H-7), δ 4.99 (2H, s, H-8), δ 0.11 (6H, s, 2xCH₃), δ 0.96 (9H, s, 3xCH₃), δ 2.30 (2H, t, 7.6 Hz, H-2'), δ 1.60 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), δ 0.84 (3H, t, 6.8 Hz, H-13'). ¹³C-NMR (CDCl₃, 100.576 MHz): δ 150.8 (s, C-4), 145.0 (s, C-3), δ 112.4 (d, C-2), δ 129.4 (s, C-1), δ 121.0 (d, C-6), δ 120.6 (d, C-5), δ 55.3 (q, C-7), δ 66.0 (t, C-8), δ 18.3 (s, C-9), δ -4.8 (2C, q, C-10), δ 25.6 (3C, q, C-11), δ 173. (s, C-1'), δ 34.3 (t, C-2'), δ 24.9 (t, C-3'), δ 29.0 (t, C-4'), δ 29.2 (t, C-5'), δ 29.5 (t, C-6'), δ 29.5 (t, C-7'), δ 29.4 (t, C-8'), δ 29.5 (t, C-9'), δ 29.2 (t, C-10'), δ 31.8 (t, C-11'), δ 22.6 (t, C-12'), δ 14.0 (q, C-13').

*4-tert-butyldimethylsilyloxy-3-methoxybenzyl tetradecanoate*: ¹H-NMR (CDCl₃, 399.945 MHz): δ 6.84 (1H, s^{b}, H-2), δ 6.80 (1H, d^{b}, H-6), δ 6.80 (1H, d^{b}, H-5), δ 3.79 (3H, s, H-7), δ 5.02 (2H, s, H-8), δ 0.15 (6H, s, 2xCH₃), δ 0.98 (9H, s, 3xCH₃), δ 2.33 (2H, t, 7.6 Hz, H-2'), δ 1.63 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), δ 1.25 (2H, m, H-13'), δ 0.87 (3H, t, 6.8 Hz, H-14'). ¹³C-NMR (CDCl₃, 100.576 MHz) : δ 150.8 (s, C-4), δ 145.0 (s, C-3), δ 112.4 (d, C-2), δ 129.5 (s, C-1), δ 121.7 (d, C-6), δ 120.7 (d, C-5), δ 55.3 (q, C-7), δ 66.1 (t, C-8), δ 18.3 (s, C-9), δ -4.7 (2C, q, C-10), δ 25. (3C, q, C-11), δ 173.6 (s, C-1'), δ 34.3 (t, C-2'), δ 24.9 (t, C-3'), δ 29.1 (t, C-4'), δ 29.2 (t, C-5'), δ 29.4 (t, C-6'), δ 29.5 (t, C-7'), δ 29.6 (t, C-8'), δ 29.6 (t, C-9'), δ 29.6 (t, C-10'), δ 29.3 (t, C-11'), δ 31.9 (t, C-12'), δ 22.6 (t, C-13'), δ 14.0 (q, C-14').

*4-tert-butyldimethylsilyloxy-3-methoxybenzyl hexadecanoate*: ¹H-NMR (CDCl₃, 399.945 MHz) : δ 6.84 (1H, s^{b}, H-2), δ 6.80 (1H, d^{b}, H-6), δ 6.80 (1H, d^{b}, H-5), δ 3.79 (3H, s, H-7), δ 5.01 (2H, s, H-8), δ 0.15 (6H, s, 2xCH₃), δ 0.98 (9H, s, 3xCH₃), δ 2.31 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), δ 1.25 (2H, m, H-13'), δ 1.25 (2H, m, H-14'), δ 1.25 (2H, m, H-15'), δ 0.86 (3H, t, 6.8 Hz, H-16'). ¹³C-NMR (CDCl₃, 100.576 MHz): δ 150.8 δ (s, C-4), δ 145.0 (s, C-3), δ 112.4 (d, C-2) δ 129.5 (s, C-1), δ 121.1 (d, C-6), δ 120.7 (d, C-5), δ 55.3 (q, C-7), δ 66.1 (t, C-8), δ 18.3 (s, C-9), δ -4.7 (2C, q, C-10), δ 25.6 (3C, q, C-11), δ 173.7 (s, C-1'), δ 34.3 (t, C-2'), δ 24.9 (t, C-3'), δ 29.1 (t, C-4'), δ 29.2 (t, C-5'), δ 29. (t, C-6'), δ 29.5 (t, C-7'), δ 29.6 (t, C-8'), δ 29.6 (t, C-9'), δ 29.6 (t, C-10'),δ 29.6 (t, C-11'), δ 29.6 (t, C-12'), δ 29.3 (t, C-13'), δ 31.9 (t, C-14'), δ 22.6 (t, C-15'), δ 14.0 (q, C-16').

*4-tert-butyldimethylsilyloxy-3-methoxybenzyl 8-methylnonanoate*: ¹H-NMR (CDCl₃, 399.945 MHz) : δ 6.83 (1H, s^{b}, H-2), δ 6.80 (1H, d^{b}, H-6), δ 6.80 (1H, d^{b}, H-5), δ 3.79 (3H, s, H-7), δ 5.02 (2H, s, H-8), δ 0.14 (6H, s, 2xCH₃), δ 0.98 (9H, s, 3xCH₃), δ 2.32 (2H, t, 7.6 Hz, H-2'), δ 1.62 (2H, quin, 7.6, 7.6 Hz, H-3'), δ 1.25 (2H, m, H-4'), δ 1.25 (2H, m, H-5'), δ 1.25 (2H, m, H-6'), δ 1.13 (2H, quin, 6.4 Hz, H-7'), δ 1.49 (1H, m, 6.4, 6.4 Hz, H-8'), δ 0.85 (6H, d, 6.4 Hz, H-9'-10'). ¹³C-NMR (CDCl₃, 100.576 MHz): δ 150.9 (s, C-4), δ 145.1 (s, C-3), δ 112.5 (d, C-2), δ 129.5 (s, C-1), δ 121.1 (d, C-6), δ 120.7 (d, C-5), δ 55.4 (q, C-7), δ 66.2 (t, C-8), δ 18.4 (s, C-9), δ -4.7 (2C, q, C-10), δ 25.7 (3C, q, C-11), δ 173.8 (s, C-1'), δ 34.4 (t, C-2'), δ 25.0 (t, C-3'), δ 29.5 (t, C-4'), δ 29.1 (t, C-5'), δ 27.2 (t, C-6'), δ 38.9 (t, C-7'), δ 27.9 (d, C-8'), δ 22.6 (2C, q, C-9'-10').

*4-tert-butyldimethylsilyloxy-3-methoxybenzyl (E)-8-methyl-6-nonanoate*: ¹H-NMR (CDCl₃, 399.945 MHz) : δ 6.83 (1H, s^{b}, H-2), δ 6.80 (1H, d^{b}, H-6), δ 6.80 (1H, d^{b}, H-5), δ 3.79 (3H, s, H-7), δ 5.02 (2H, s, H-8), δ 0.14 (6H, s, 2xCH₃), δ 0.98 (9H, s, 3xCH₃), δ 2.33 (2H, t, 7.5 Hz, H-2'), δ 1.63 (2H, quin, 7. 5, 7.8 Hz, H-3'), δ 1.36 (2H, quin, 7.8, 7.6 Hz, H-4'), δ 1.97 (2H, td, 7.6, 6.1 Hz, H-5'), δ 5.29 (1H, dt, 6.1, 15.2 Hz, H-6'), δ 5.36 (1H, quin, 15.2, 5.9 Hz, H-7'), δ 2.20 (1H, dh, 5.9, 6.7 Hz, H-8'), δ 0.94 (6H, d, 6.7 Hz, H-9'-10'). ¹³C-NMR (CDCl₃, 100.576 MHz) : δ 150.9 (s, C-4), δ 145.1 (s, C-3), δ 112.4 (d, C-2), δ 129.3 (s, C-1), δ 121.1 (d, C-6), δ 120.7 (d, C-5), δ 55.4 (q, C-7), δ 66.2 (t, C-8), δ 18.4 (s, C-9), δ -4.7 (2C, q, C-10), δ 25.7 (3C, q, C-11), δ 173.6 (s, C-1'), δ 34.2 (t, C-2'), δ 24.4 (t, C-3'), δ 29.1 (t, C-4'), δ 32.1 (t, C-5'), δ 126.4 (d, C-6'), δ 138.0 (d, C-7'), δ 30.9 (d, C-8'), δ 22.6 (2C, q, C-9'-10').

### Step 4: Desilylating silylated capsinoids.

The last step required for the complete synthesis of the capsinoids is the desilylation of the previously formed compounds (V). To that end the silylated capsinoids are reacted with a 0.25 M HCl / ethanol mixture at a 1:5 ratio. Deprotection is thus achieved without observing the breakage of the ester bond.

The silylated capsinoids (V) are introduced in a 250 mL round-bottom flask and 80 mL of the 0.25M HCl / ethanol mixture (1:5) are added. Inert argon atmosphere is introduced and the reaction mixture is maintained under stirring for 18 hours. The reaction is followed by means of TLC (eluent: 20% ethyl acetate, 80% hexane; stain: anisaldehyde).

Once the reaction has ended, it is stopped with brine. The aqueous phase is extracted 3 times with ethyl acetate (3x100 mL). The three organic phases are pooled, dried with anhydrous MgSO₄ and concentrated under reduced pressure to remove the ethyl acetate (room temperature). The obtained oil is dissolved in a small amount of ethyl acetate and silica gel is added to form the head of the column for the purification of the end product. The ethyl acetate is evaporated under reduced pressure (room temperature).

The chromatographic separation is carried out with silica gel and the polarity of the eluent is 15% ethyl acetate in hexane. The chromatographic separation is followed by TLC (eluent: 20% ethyl acetate, 80% hexane; stain: anisaldehyde). Finally a yellowish oil corresponding to the capsinoids (I) is obtained (yields 77-87%).

### Characterizing the silylated capsinoids:

*4-hydroxy-3-methoxybenzyl ethanoate*: ¹H-NMR (CDCl₃, 399.945 MHz): δ 6.86 (1H, d, 1.8 Hz, H-2), δ 6.85 (1H, dd, 1.8, 6.4 Hz, H-6), δ 6.88 (1H, d, 6.4 Hz, H-5), δ 3.88 (3H, s, H-7), δ 5.01 (2H, s, H-8), δ 5.79 (1H, s, OH), δ 2.07 (3H, s, H-2'). ¹³C-NMR (CDCl₃, 100.576 MHz): δ 145.8 (s, C-4), δ 146.5 (s, C-3), δ 111.3 (d, C-2), δ 127.7 (s, C-1), δ 122.0 (d, C-6), δ 114.3 (d, C-5), δ 55.9 (q, C-7), δ 66.5 (t, C-8), δ 171. (s, C-1'), δ 21.0 (q, C-2'). UV λₘₐₓ 279 nm.

*4-hydroxy-3-methoxybenzyl propanoate*: ¹H-NMR (CDCl₃, 399.945 MHz): δ 6.84 (1H, d, 1.8 Hz, H-2), δ 6.83 (1H, dd, 1.8, 7.3 Hz, H-6), δ 6.86 (1H, d, 7.3 Hz, H-5), δ 3.84 (3H, s, H-7), δ 5.00 (2H, s, H-8), δ 5.94 (1H, s, OH), δ 2.33 (2H, q, 7.6 Hz, H-2'), δ 1.11 (3H, t, 7.6 Hz, H-3'). ¹³C-NMR (CDCl₃, 100.576 MHz): δ 145.7 (s, C-4), δ 146.5 (s, C-3), δ 111.2 (d, C-2), δ 127.8 (s, C-1), δ 121.8 (d, C-6), δ 114.3 (d, C-5), δ 55.7 (q, C-7), δ 66.2 (t, C-8), δ 174.3 (s, C-1'), δ 27.5 (t, C-2'), δ 8.9 (q, C-3'). UV λₘₐₓ 279 nm.

*4-hydroxy-3-methoxybenzyl butanoate*: ¹H-NMR (CDCl₃, 399.945 MHz): δ 6.82 (1H, d, 1.8 Hz, H-2), δ 6.81 (1H, dd, 1.8, 7.6 Hz, H-6), δ 6.84 (1H, d, 7.6 Hz, H-5), δ 3.82 (3H, s, H-7), δ 4.98 (2H, s, H-8), δ 5.81 (1H, s, OH), δ 2.27 (2H, t, 7.6 Hz, H-2'), δ 11.61 (2H, tq, 7.6, 7.3 Hz, H-3'), δ 0.88 (3H, t, 7.3 Hz, H-4'). ¹³C-NMR (CDCl₃, 100.576 MHz) : δ 145.7 (s, C-4), δ 146.5 (s, C-3), δ 111.2 (d, C-2), δ 127.8 (s, C-1), δ 121.7 (d, C-6), δ 114.3 (d, C-5), δ 55.7 (q, C-7), δ 66.1 (t, C-8), δ 173.5 (s, C-1'), δ 36.0 (t, C-2'), δ 18.2 (t, C-3'), δ 13.4 (q, C-4'). UV λₘₐₓ 279 nm.

*4-hydroxy-3-methoxybenzyl pentanoate*: ¹H-NMR (CDCl₃, 399.945 MHz) : δ 6.85 (1H, d, 1.8 Hz, H-2), δ 6.84 (1H, dd, 1.8, 7.3 Hz, H-6), δ 6.87 (1H, d, 7.3 Hz, H-5), δ 3.85 (3H, s, H-7), δ 5.01 (2H, s, H-8), δ 5.95 (1H, s, OH), δ 2.32 (2H, t, 7.6 Hz, H-2'), δ 1.60 (2H, quin, 7.6, 7.2 Hz, H-3'), δ 1.32 (2H, tq, 7.2, 7.2 Hz, H-4'), δ 0.88 (3H, t, 7.2 Hz, H-5'). ¹³C-NMR (CDCl₃, 100.576 MHz): δ 145.7 (s, C-4), δ 146.5 (s, C-3), δ 111.2 (d, C-2), δ 127.8 (s, C-1), δ 121.8 (d, C-6), δ 114.3 (d, C-5), δ 55.7 (q, C-7), δ 66.2 (t, C-8), δ 173.7 (s, C-1'), δ 33.9 (t, C-2'), δ 26.9 (t, C-3'), δ 22.1 1 (t, C-4'), δ 13.5 (q, C-5'). UV λₘₐₓ 279 nm.

*4-hydroxy-3-methoxybenzyl hexanoate*: ¹H-NMR (CDCl₃, 399.945 MHz): δ 6.82 (1H, d, 1.8 Hz, H-2), δ 6.81 (1H, dd, 1.8, 6.7 Hz, H-6), δ 6.84 (1H, d, 6.7 Hz, H-5), δ 3.86 (3H, s, H-7), δ 5.02 (2H, s, H-8), δ 5.85 (1H, s, OH), δ 2.32 (2H, t, 7.6 Hz, H-2'), δ 1.62 (2H, quin, 7.6, 7.6 Hz, H-3'), δ 1.27 (2H, m, 7.6, 7.2 Hz, H-4'), δ 1.27 (2H, m, 7.2, 7.2 Hz, H-5'), δ 0.87 (3H, t, 7.2 Hz, H-6'). ¹³C-NMR (CDCl₃, 100.576 MHz) : δ 145.7 (s, C-4), 146.4 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.2 (t, C-2'), δ 24.5 (t, C-3'), δ 31.2 (t, C-4'), δ 22.2 (t, C-5'), δ 13.8 (q, C-6'). UV λₘₐₓ 279 nm.

*4-hydroxy-3-methoxybenzyl heptanoate*: ¹H-NMR (CDCl₃, 399.945 MHz): δ 6.85 (1H, d, 1.8 Hz, H-2), δ 6.84 (1H, dd, 1.8, 7.0 Hz, H-6), δ 6.87 (1H, d, 7.0 Hz, H-5), δ 3.85 (3H, s, H-7), δ 5.01 (2H, s, H-8), δ 6.03 (1H, s, OH), δ 2.31 (2H, t, 7.6 Hz, H-2'), δ 1. 60 (2H, quin, 7.6, 7.6 Hz, H-3'), δ 1.25 (2H, m, H-4'), δ 1.25 (2H, m, H-5'), δ 1.25 (2H, m, H-6'), δ 0.85 (3H, t, 7.2 Hz, H-7'). ¹³C-NMR (CDCl₃, 100.576 MHz) : δ 145. (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ 24.8 (t, C-3'), δ 28.7 (t, C-4'), δ 31.3 (t, C-5'), δ 22.4 (t, C-6'), δ 13.9 (q, C-7'). UV λₘₐₓ 279 nm.

*4-hydroxy-3-methoxybenzyl octanoate*: ¹H-NMR (CDCl₃, 399.945 MHz): δ 6.86 (1H, d, 1.8 Hz, H-2), δ 6.85 (1H, dd, 1.8, 6.7 Hz, H-6), δ 6.88 (1H, d, 6.7 Hz, H-5), δ 3.85 (3H, s, H-7), δ 5.01 (2H, s, H-8), δ 6.30 (1H, s, OH), δ 2.31 (2H, t, 7.6 Hz, H-2'), δ 1.61 (2H, quin, 7.6, 7.6 Hz, H-3'), δ 1.25 (2H, m, H-4'), δ 1.25 (2H, m, H-5'), δ 1.25 (2H, m, H-6'), δ 1.25 (2H, m, H-7'), δ 0.85 (3H, t, 6.8 Hz, H-8'). ¹³C-NMR (CDCl₃, 100.576 MHz): v 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8),δ 173.8 (s, C-1'), δ 34.3 (t, C-2'), δ 24.9 (t, C-3'), δ 28.9 (t, C-4'), δ 28.8 (t, C-5'), δ 31.5 (t, C-6'), δ 22.5 (t, C-7'), δ 13.9 (q, C-8'). UV λₘₐₓ 279 nm.

*4-hydroxy-3-methoxybenzyl nonanoate*: ¹H-NMR (CDCl₃, 399.945 MHz): δ 6.85 (1H, d, 1.8 Hz, H-2), δ 6.84 (1H, dd, 1.8, 7.0 Hz, H-6), δ 6.87 (1H, d, 7.0 Hz, H-5), δ 3.86 (3H, s, H-7), δ 5.01 (2H, s, H-8), δ 6.06 (1H, s, OH), δ 2.31 (2H, t, 7.6 Hz, H-2'), δ 1.61 (2H, quin, 7.6, 7.6 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 0.85 (3H, t, 6.8 Hz, H-9'). ¹³C-NMR (CDCl₃, 100.576 MHz): δ 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173. δ (s, C-1'), δ 34.3 (t, C-2'), δ 24.9 (t, C-3'), δ 29.0 (t, C-4'), δ 29.1 (t, C-5'), δ 29.0 (t, C-6'), δ 31.7 (t, C-7'), δ 22.5 (t, C-8'), δ 14.0 (q, C-9'). UV λₘₐₓ 279 nm.

*4-hydroxy-3-methoxybenzyl decanoate*: ¹H-NMR (CDCl₃, 399.945 MHz): δ 6.85 (1H, d, 1.8 Hz, H-2), δ 6.84 4 (1H, dd, 1.8, 6.4 Hz, H-6), δ 6.87 (1H, d, 6.4 Hz, H-5), δ 3.86 (3H, s, H-7), δ 5.01 (2H, s, H-8), δ 5.76 (1H, s, OH), δ 2.31 (2H, t, 7.6 Hz, H-2'), δ 1.61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 0.86 (3H, t, 7.2 Hz, H-10'). ¹³C-NMR (CDCl₃, 100.576 MHz) : δ 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ24.9 (t, C-3'), δ 29.0 (t, C-4'), δ 29.2 (t, C-5'), δ 29.3 (t, C-6'), δ 29.2 (t, C-7'), δ 31.8 (t, C-8'), δ 22.6 (t, C-9'), δ 14.0 (q, C-10'). UV λₘₐₓ 279 nm.

*4-hydroxy-3-methoxybenzyl undecanoate*: ¹H-NMR (CDCl₃, 399. 945 MHz) : δ 6.86 (1H, d, 1.8 Hz, H-2), δ 6.85 (1H, dd, 1.8, 6.7 Hz, H-6), δ 6.88 (1H, d, 6.7 Hz, H-5), δ 3.88 (3H, s, H-7), δ 5.01 (2H, s, H-8), δ 5.72 (1H, s, OH), δ 2.31 (2H, t, 7.6 Hz, H-2'), δ 1. 61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 0.86 (3H, t, 7.0 Hz, H-11'). ¹³C-NMR (CDCl₃, 100.576 MHz): δ 145.7 (s, C-4), δ 146.4 (s, C-3), δ 111.2 (d, C-2), δ 128.0 (s, C-1), δ 121.9 (d, C-6), δ 114.3 (d, C-5), δ 55.9 (q, C-7), δ 66.2 (t, C-8), δ 173.8 (s, C-1'), δ 34.3 (t, C-2'), δ 24.9 (t, C-3'), δ 29.1 (t, C-4'), δ 29.2 (t, C-5'), δ 29.5 (t, C-6'), δ 29.4 (t, C-7'), δ 29.2 (t, C-8'), δ 31.8 (t, C-9'), δ 22.6 (t, C-10'), δ 14.0 (q, C-11'). UV λₘₐₓ 279 nm.

*4-hydroxy-3-methoxybenzyl dodecanoate*: ¹H-NMR (CDCl₃, 399. 945 MHz) : δ 6.86 (1H, d, 1.8 Hz, H-2), δ 6.85 (1H, dd, 1.8, 6.1 Hz, H-6), δ 6.88 (1H, d, 6.1 Hz, H-5), δ 3.87 (3H, s, H-7), δ 5.02 (2H, s, H-8), δ 5.78 (1H, s, OH), δ 2.31 (2H, t, 7.6 Hz, H-2'), δ 1. 61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 0.87 (3H, t, 6.8 Hz, H-12'). ¹³C-NMR (CDCl₃, 100.576 MHz): δ 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ 24.9 (t, C-3'), δ 29.1 (t, C-4'), δ 29.2 (t, C-5'), δ 29.5 (t, C-6'), δ 29.5 (t, C-7'), δ 29.4 (t, C-8'), δ 29.3 (t, C-9'), δ 31.8 (t, C-10'), δ 22.6 (t, C-11'), δ 14.0 (q, C-12'). UV λₘₐₓ 279 nm.

*4-hydroxy-3-methoxybenzyl tridecanoate*: ¹H-NMR (CDCl₃, 399.945 MHz): δ 6.86 (1H, d, 1.8 Hz, H-2), δ 6.85 1H, dd, 1.8, 6.7 Hz, H-6), δ 6.88 (1H, d, 6.7 Hz, H-5), δ 3.87 (3H, s, H-7), δ 5.02 (2H, s, H-8), δ 6.49 (1H, s, OH), δ 2.32 (2H, t, 7.6 Hz, H-2'), δ 1.62 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), δ 0.87 (3H, t, 6.8 Hz, H-13'). ¹³C-NMR (CDCl₃, 100.576 MHz) : δ 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ 24.9 (t, C-3'), δ 29.1 (t, C-4'), δ 29.2 (t, C-5'), δ 29.4 (t, C-6'), δ 29.5 (t, C-7'), δ 29.6 (t, C-8'), δ 29.5 (t, C-9'), δ 29.3 (t, C-10'), δ 31.8 (t, C-11'), δ 22.6 6 (t, C-12'), δ 14.0 (q, C-13'). UV λₘₐₓ 279 nm.

*4-hydroxy-3-methoxybenzyl tetradecanoate*: ¹H-NMR (CDCl₃, 399.945 MHz): δ 6.86 (1H, d, 1.8 Hz, H-2), δ 6.85 (1H, dd, 1.8, 5.6 Hz, H-6), δ 6.88 (1H, d, 5.6 Hz, H-5), δ 3.87 (3H, s, H-7), δ 5.02 (2H, s, H-8), δ 5.75 (1H, s, OH), δ 2.31 (2H, t, 7.6 Hz, H-2'), δ 1. 61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), 1.25 (2H, m, H-13'), δ 0.87 (3H, t, 6.8 Hz, H-14'). ¹³C-NMR (CDCl₃, 100.576 MHz): δ 145.7 (s, C-4), δ 146.4 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2"), δ 24.9 (t, C-3'), δ 29.1 (t, C-4'), δ 29.2 (t, C-5'), δ 29.4 (t, C-6'), δ 29.5 (t, C-7'), δ 29.6 (t, C-8'), δ 29.6 (t, C-9'), δ 29.6 (t, C-10'), δ 29.3 (t, C-11'), δ 31.9 (t, C-12'), δ 22.6 (t, C-13'), δ 14.0 (q, C-14'). UV λₘₐₓ 279 nm.

*4-hydroxy-3-methoxybenzyl hexadecanoate*: ¹H-NMR (CDCl₃, 399. 945 MHz) : δ 6.85 (1H, d, 1.8 Hz, H-2), δ 6.84 (1H, dd, 1.8, 5.9 Hz, H-6), δ 6.87 (1H, d, 5.9 Hz, H-5), δ 3.87 (3H, s, H-7), δ 5.01 (2H, s, H-8), δ 5.77 (1H, s, OH), δ 2.31 (2H, t, 7.6 Hz, H-2'), δ 1. 61 (2H, quin, 7.6, 7.2 Hz, H-3'), δ 1.25 (2H, m, H-4'), δ 1.25 (2H, m, H-5'), δ 1.25 (2H, m, H-6'), δ 1.25 (2H, m, H-7'), δ 1.25 (2H, m, H-8'), δ 1.25 (2H, m, H-9'), δ 1.25 (2H, m, H-10'), δ 1.25 (2H, m, H-11'), δ 1.25 (2H, m, H-12'), 1.25 (2H, m, H-13'), δ 1.25 (2H, m, H-14'), 1.25 (2H, m, H-15'), δ 0.86 (3H, t, 6.8 Hz, H-16'). ¹³C-NMR (CDCl₃, 100.576 MHz) : δ 145.7 (s, C-4), δ 146.5 (s, C-3), δ 111.2 (d, C-2), δ 128.0 (s, C-1), δ 121.9 (d, C-6), δ 114.3 (d, C-5), δ 55.9 (q, C-7), δ 66.2 (t, C-8), δ 173.8 (s, C-1'), δ 34.3 (t, C-2'), δ 24.9 (t, C-3'), δ 29.0 (t, C-4'), δ 29.1 (t, C-5'), δ 29.3 (t, C-6'), δ 29.4 (t, C-7'), δ 29.5 (t, C-8'), δ 29.6 (t, C-9'), δ 29.6 (t, C-10'), δ 29.6 (t, C-11'), δ 29.6 (t, C-12'), δ 29.2 (t, C-13'), δ 31.9 (t, C-14'), δ 22.6 (t, C-15'), δ 14.0 (q, C-16'). UV λₘₐₓ 279 nm.

*4-hydroxy-3-methoxybenzyl 8-methylnonanoate (dihydrocapsaicin)*: ¹H-NMR (CDCl₃, 399. 945 MHz) : δ 6.86 (1H, d, 1.8 Hz, H-2), δ 6.85 (1H, dd, 1.8, 5.9 Hz, H-6), δ 6.88 (1H, d, 5.9 Hz, H-5), δ 3.87 (3H, s, H-7), δ 5.02 (2H, s, H-8), δ 5.78 (1H, s, OH), δ 2.32 (2H, t, 7.6 Hz, H-2'), δ 1.62 (2H, quin, 7.6, 7.6 Hz, H-3'), δ 1.25 (2H, m, H-4'), δ 1.25 (2H, m, H-5'), δ 1.25 (2H, m, H-6'), δ 1.12 (2H, quin, 6. 4 Hz, H-7'), δ 1.49 (1H, m, 6.4, 6.4 Hz, H-8'), δ 0.84 (6H, d, 6.4 Hz, H-9'-10'). ¹³C-NMR (CDCl₃, 100.576 MHz) : δ 145.7 (s, C-4), δ 146.5 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ 24.9 (t, C-3'), δ 29.4 (t, C-4'), δ 29.1 (t, C-5'), δ 27.1 (t, C-6'), δ 38.9 (t, C-7'), δ 27.9 (d, C-8'), δ 22.5 (2C, q, C-9'-10'). UV λₘₐₓ 279 nm.

*4-Hydroxy-3-methoxybenzyl (E)-8-methyl-6-nonanoate (capsaicin)*: ¹H-NMR (CDCl₃, 399. 945 MHz) : δ 6.84 (1H, d, 1.8 Hz, H-2), δ 6.83 (1H, dd, 1.8, 7.2 Hz, H-6), δ 6.86 (1H, d, 7.2 Hz, H-5), δ 3.86 (3H, s, H-7), δ 5.01 (2H, s, H-8), δ 5.67 (1H, s, OH), δ 2.34 (2H, t, 7.5 Hz, H-2'), δ 1.64 (2H, quin, 7.5, 7.8 Hz, H-3'), δ 1.38 (2H, quin, 7.8, 7.6 Hz, H-4'), δ 1.98 (2H, dt, 7.6, 6.1 Hz, H-5'), δ 5.30 (H, dt, 15.2, 6.1 Hz, H-6'), δ 5.37 (1H, dd, 15.2, 5.9 Hz, H-7'), δ 2.21 (1H, dh, 5. 9, 6. 7 Hz, H-8'), δ 0.95 (6H, d, 6.7 Hz, H-9'-10'). ¹³C-NMR (CDCl₃, 100.576 MHz): δ 145.7 (s, C-4), δ 146.4 (s, C-3), δ 111.2 (d, C-2), δ 127.9 (s, C-1), δ 121.9 (d, C-6), δ 114.3 (d, C-5), δ 55.8 (q, C-7), δ 66.2 (t, C-8), δ 173.8 (s, C-1'), δ 34.3 (t, C-2'), δ 24.5 (t, C-3'), δ 29.1 (t, C-4'), δ 32.1 (t, C-5'), δ 126.4 (d, C-6'), δ 138.1 (d, C-7'), δ 30.9 (d, C-8'), δ 22.6 (2C, q, C-9'-10'). UV λₘₐₓ 279 nm.

## Claims

1. A method for the chemical synthesis of capsinoids comprising the protection of the phenol hydroxyl group of the alcohol resulting from the reduction of vanillin, prior to the esterification of the primary hydroxyl group with the suitable acyl moiety.

2. The method for the chemical synthesis of capsinoids according to claim 1, starting from vanillin comprising the protection of the alcohol, reduction of the aldehyde, esterification and deprotection.

3. The method for the chemical synthesis of capsinoids according to claims 1 and 2, **characterized by** obtaining the protected vanillin with a protective group which prevents the esterification of hydroxyl.

4. The method for the chemical synthesis of capsinoids according to claims 1 to 3, **characterized by** the reduction of the carbonyl of the protected vanillin to obtain a primary alcohol without the removal of the protective group.

5. The method for the chemical synthesis of capsinoids according to claims 1 to 4, **characterized by** the esterification of the reduced and protected vanillin with acyl chlorides or any other acylating agent.

6. The method for the chemical synthesis of capsinoids according to claims 1 to 5, **characterized by** the deprotection of the protected capsinoids obtained giving rise to the capsinoids.

7. Compounds with the structure I, obtained according to claims 1 to 6:
4-hydroxy-3-methoxybenzyl propanoate (R = -CH₂CH₃).
4-hydroxy-3-methoxybenzyl butanoate (R = -CH₂CH₂CH₃).
4-hydroxy-3-methoxybenzyl pentanoate (R = -(CH₂)₃-CH₃).
4-hydroxy-3-methoxybenzyl tridecanoate (R = (CH₂)₁₁-CH₃).
